# EUROPEAN PATENT APPLICATION

(11) **EP 3 480 823 A1**
(43) Date of publication of application: **08.05.2019**
(21) Application number: 17199773.7
(22) Date of filing: 02.11.2017
(51) Int. Cl.: G16H 50/20

(54) **CLINICAL DECISION SUPPORT**

(71) Applicant: Koninklijke Philips N.V., 5656 AE Eindhoven (NL)
(72) Inventor: WIMBERGER-FRIEDL, Reinhold, 5656 AE Eindhoven (NL); VITTORINO DE ALMEIDA, Vanda Lucia de Carvalho, 5656 AE Eindhoven (NL); DA SILVA RODRIGUES, Pedro Jorge, 5656 AE Eindhoven (NL); BUCUR, Anca Ioana Daniela, 5656 AE Eindhoven (NL)
(74) Representative: de Haan, Poul Erik

(57) **Abstract**

A clinical decision support system (100) is disclosed. The clinical decision support system (100) comprises a processor (102) configured to: establish a subject profile for defining a subject according to a plurality of features; identify a set of clinical trials that are relevant to the subject, wherein each clinical trial in the set of relevant clinical trials corresponds to at least one therapy has inclusion criteria that are satisfied by features of the plurality of features of the subject profile; rank the therapies corresponding to the relevant clinical trials; and output the ranking of the therapies for the subject. A method and a computer program product are also disclosed.

## Description

### FIELD OF THE INVENTION

The invention relates to clinical decision support and, more particularly, to a system and method for ranking therapies relevant to a subject.

### BACKGROUND OF THE INVENTION

When a clinician is required to select a therapy to be provided to a subject, clinical guidelines or clinical pathways are typically used to aid the clinician's decision making. The chosen therapy may be provided to the subject over a period of time during which time the response of the subject to the therapy may be monitored. With some diseases, such as cancer, therapy may lead to an increase in resistance of the disease to treatment, and may lead to progression (i.e. worsening) of the disease.

For some diseases, first and second therapies (referred to as first line and second line therapies) may be unsuccessful and, as a result, subsequent therapies (e.g. a third line therapy) may have little or no curative intent, but rather may be intended to extend the life expectancy with a reasonable quality, or reduce symptoms that affect the quality of life without expending life expectancy. In such scenarios, clinical guidelines and clinical pathways offer little assistance as there is often no recognized standard care path. A clinician may, therefore, select a therapy for the subject based on his or her own knowledge, or based on scientific literature.

Clinical trials are carried out to investigate the effects that various new or adapted therapies have on different people, with an end goal to improve standard of care. Clinical trials may include multiple arms, with each arm including a group of participants receiving a particular therapy. Some arms of a clinical trial may involve a combination of therapies being provided to a single group of participants. With so many clinical trials taking place, and the large number of possible combinations of therapies, it may be difficult for a clinician to remain aware of the various therapeutic options available and suitable for their subjects.

Such problems are particularly prevalent in the field of immunotherapy for use in cancer treatment. Due to the large number of new immunotherapeutic options being developed, the number of clinical trials in this area has rapidly increased.

Therefore, there is a need for a system that enables a clinician to make a more informed decision about which therapy or therapies might be suitable for their subjects.

### SUMMARY OF THE INVENTION

According to an aspect of the invention, there is provided a clinical decision support system comprising a processor. The processor is configured to establish a subject profile for defining a subject according to a plurality of features; identify a set of clinical trials that are relevant to the subject, wherein each clinical trial in the set of relevant clinical trials corresponds to at least one therapy and has inclusion criteria that are satisfied by features of the plurality of features of the subject profile; rank the therapies corresponding to the relevant clinical trials; and output the ranking of the therapies for the subject. Such a system may provide a user (e.g. a clinician or a subject) with a list of relevant therapies for the subject based on clinical trials which may be running, or may have been successfully completed, and which the subject may have been eligible for. By evaluating the therapies corresponding to such clinical trials, a clinician may gain a greater understanding or awareness of possible therapeutic options which may be suitable for the subject.

In some embodiments, in order to rank the therapies, the processor may be configured to rank the identified set of clinical trials based on clinical benefits achieved by each clinical trial.

The processor may be configured to rank the identified set of clinical trials according to at least one of: a similarity of features of the subject to corresponding features of participants of the clinical trials; and a level of information available regarding the clinical trials.

The clinical benefits achieved by a clinical trial may be defined by at least one of: a measure of the progression-free survival of participants in the clinical trial; a measure of the overall survival of participants in the clinical trial; and a measure of the response rate of participants in the clinical trial.

The processor may, in some embodiments, be configured to rank the therapies based on a weighting score. The weighting score may be based on at least one of: a satisfaction score indicating a degree of satisfaction of the inclusion criteria by the plurality of features of the subject profile; an outcome score indicating an extent to which each trial achieved clinical benefits; and a supplemental effect score indicating a measure of expected side effects caused by each trial.

In some embodiments, the processor may be configured to establish the subject profile by obtaining a clinical information relating to the subject from a clinical database.

The processor may be configured to identify the set of clinical trials by searching a clinical trails database, the clinical trials database including at least clinical trials that have provided outcome data. For example, the clinical trials database may include trials that have ended.

In some embodiments, the processor may be configured to group the ranked therapies according to at least one of: biological similarities between the therapies; and similarities between features of the subject profile and features of profiles of participants in the set of clinical trials.

The processor may be configured to present the output ranked therapies to a user along with at least one of: expected benefits of each presented therapy; and potential risks associated with each presented therapy.

In some embodiments, a clinical trial may be considered to be relevant to the subject if a threshold number of inclusion criteria are satisfied by features of the subject profile.

According to another aspect of the invention, there is provided a method for ranking a plurality of therapies for a subject. The method comprises establishing a subject profile for defining the subject according to a plurality of features; identifying a set of clinical trials that are relevant to the subject, wherein each clinical trial in the set of relevant clinical trials corresponds to at least one therapy, and has inclusion criteria that are satisfied by features of the plurality of features of the subject profile; ranking the therapies corresponding to the relevant clinical trials; and outputting the ranking of the therapies for the subject.

In some embodiments, the method may further comprise applying a weighting score to each therapy. The ranking may be based on the applied weighting score. The weighting score may, in some embodiments, be based on at least one of: a satisfaction score indicating a degree of satisfaction of the inclusion criteria by the plurality of features of the subject profile; an outcome score indicating an extent to which each trial achieved clinical benefits; and a supplemental effect score indicating a measure of expected side effects caused by each trial.

According to another aspect of the invention, there is provided a computer program product comprising a non-transitory computer readable medium, the computer readable medium having computer readable code embodied therein, the computer readable code being configured such that, on execution by a suitable computer or processor, the computer or processor is caused to perform any of the methods disclosed herein.

According to another aspect of the invention, there is provided a clinical decision support system (100), comprising: a processor (102) configured to: establish a subject profile for defining a subject according to a plurality of features; identify a set of clinical trials that are relevant to the subject, wherein each clinical trial in the set of relevant clinical trials corresponds to at least one therapy, and has associated inclusion criteria, and wherein each clinical trial has inclusion criteria that match features of the plurality of features of the subject profile; rank the therapies corresponding to the relevant clinical trials; and output the ranking of the therapies for the subject.

According to yet another aspect of the invention, there is provided a device for providing clinical decision support, the device comprising: a profile unit for defining a profile of a subject based on a plurality of features; an identification unit for identifying a set of clinical trials that are relevant to the subject, wherein each clinical trial in the set of relevant clinical trials corresponds to at least one therapy, and has associated inclusion criteria, and wherein each clinical trial has inclusion criteria that match features of the plurality of features of the subject profile; a ranking unit for ranking the therapies corresponding to the relevant clinical trials; and a display for displaying the ranking of the therapies.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified schematic of an example of a system according to embodiments;
Fig. 2 is a simplified schematic of a further example of a system according to embodiments;
Fig. 3 is a flowchart of an example of a method for ranking therapies according to embodiments;
Fig. 4 is a flowchart of a further example of a method for ranking therapies according to embodiments;
Fig. 5 is a flowchart of a further example of a method for ranking therapies according to embodiments; and
Fig. 6 is a simplified schematic of a computer-readable medium and a processor.

### DETAILED DESCRIPTION OF EMBODIMENTS

The inventors have discovered that valuable information regarding the suitability of therapies for a subject (e.g. a patient) may be obtained using information on clinical trials which have taken place previously. For example, data regarding clinical trials which have ended, for which a particular subject would have been eligible, may be examined and used to decide whether a therapy (e.g. a therapy investigated as part of a previous clinical trial) is suitable for, and likely to provide a clinical benefit to, the particular subject.

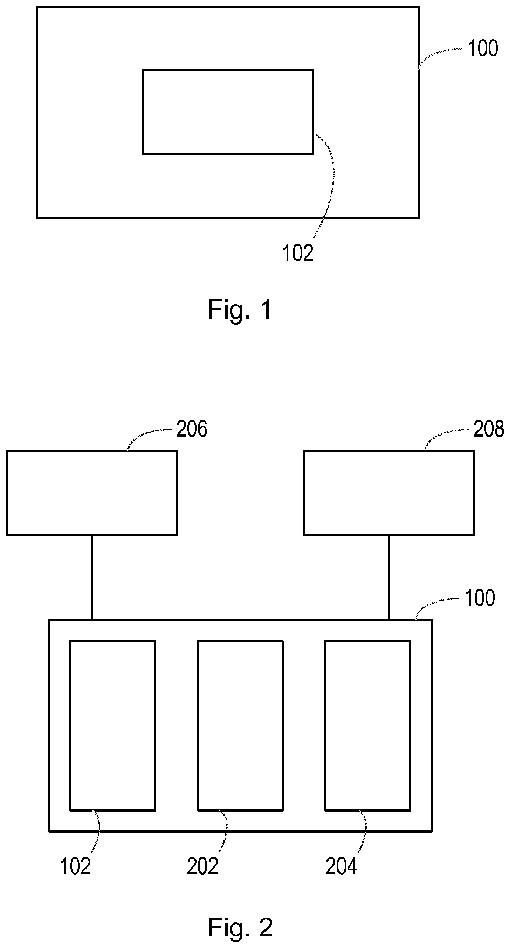

An aspect of the invention may be embodied as an apparatus or system, as shown in Fig. 1. Fig. 1 shows and example of an apparatus or system, such as a clinical support system 100, comprising a processor 102. The system 100 may comprise a computing device, such as desktop, laptop or tablet computer, a smartphone, a server, a network of computing devices, or any other apparatus or system having suitable processing functionality.

In some embodiment, the processor 102 is configured to establish a subject profile for defining a subject according to a plurality of features. A subject may, for example, be a patient who under the care of one or more clinicians or medical professionals. The subject profile may include any data relating to the subject, such as personal data (e.g. name, age and gender) relating to the subject, medical data (e.g. current and previous medical conditions, previous and current treatments and therapies, data acquired from a medical record, such as an electronic medical record (EMR), laboratory data and pathology reports) relating to the subject, or any combination of such data. The features of the subject profile may comprise one or more items of data included in the profile. For example, a feature may be that the subject is female; another feature may be that the subject is aged 54; another feature may be that the subject has previously been treated for a particular type of cancer, and so on.

In some embodiments, the processor 102 may establish the subject profile automatically, for example by assembling, interpreting and/or combining data from different records which are related to a single subject identification number. In some examples, subject or subject data may be entered manually, for example by a medical professional or clinician. In some embodiments, the processor 102 may be configured to establish the subject profile by obtaining a clinical information relating to the subject from a clinical database. Such a clinical database may form part of the system 100, or may be remote from the system, accessible by the processor 102. The clinical database may, for example, include clinical data relating to multiple subjects of a particular medical facility or medical institution or organization.

In some embodiment, the processor 102 is further configured to identify a set of clinical trials that are relevant to the subject, wherein each clinical trial in the set of relevant clinical trials corresponds to at least one therapy and has inclusion criteria that are satisfied by features of the plurality of features of the subject profile. Inclusion criteria for a clinical trial are a set of requirements that a subject must meet in order to participate in the trial. For example, inclusion criteria for a particular clinical trial may include that a participant is male, aged between 50 and 55, and is suffering from a particular medical condition. If a subject meets the criteria, then they may be eligible to participate in the clinical trial. The features included in the subject profile may be used to determine whether or not a subject is eligible for participation. If a subject is, or would be, eligible for a particular clinical trial, then that particular trial may be considered relevant to them. In some embodiments, each clinical trial may have a plurality of inclusion criteria that are satisfied by features of the plurality of features of the subject profile.

In some embodiments, a clinical trial may be relevant to the subject if a threshold number of inclusion criteria are satisfied by features of the subject profile. For example, a clinical trial may have a list of six inclusion criteria. Even though a subject who does not meet all six inclusion criteria may not be eligible for participation in the trial, the clinical trial may be considered relevant to the subject if the subject meets a defined threshold number (e.g. five) of the six inclusion criteria. Thus, a clinical trial may be considered relevant to a subject, and the therapy may be considered suitable for the subject, if only one, or very few criteria are not met, or if the subject fails to meet one or more criteria by a small defined margin. In some embodiments, exclusion criteria may be set, such that a subject is unable to participate in a particular trial if they fall within the exclusion criteria. Weights may be applied to inclusion criteria, in some embodiments. For example, some criteria may be weighted more heavily if it is considered that those criteria are of more importance (e.g. if it is considered more important that those criteria are met than other, less important criteria). In some embodiments, the relevance of a clinical trial to a subject may be based on other factors, which may be included in the subject profile or may be taken into account in some other way.

Within the set of clinical trials identified by the processor 102, each clinical trial corresponds to at least one therapy. In some embodiments, a clinical trial may correspond to multiple therapies. One or more therapies whose effectiveness is under trial may be considered to correspond to a clinical trial.

The processor 102 is further configured to rank the therapies corresponding to the relevant clinical trials. In other words, any therapies that correspond to clinical trials in the identified set of clinical trials may be listed and ordered. For example, the therapies may be ranked or ordered according to one or more defined rules or criteria, as discussed below.

The processor 102 is further configured to output the ranking of the therapies for the subject. For example, the processor 102 may output data indicating the ranking of the therapies and/or may output the list of therapies in the ranked order. In some embodiments, the ranking may be output for presentation. For example, the ranking may be output to a display device, such as a display screen for presentation to a user. In this way, a clinician may use the clinical support system 100 to obtain a ranked list of possible therapies that correspond to clinical trials which are relevant to their subject. The ranked list may, for example, be used by the clinician to evaluate which therapies are most likely to assist their subject, due to the relevance of the clinical trials for which the subject may be (or may have been) eligible, but about which the clinician (and/or the subject) was not aware.

The clinical trials in the set of clinical trials identified by the processor 102 may be trials which are ongoing at the time that they are identified. However, while a clinical trial is ongoing, there is likely to be little information published in the way of success rates or effectiveness of the therapies under trial. In some embodiments, the clinical trials identified by the processor 102 may be trials which have taken place in the past, and which have completed, been terminated, or have otherwise ended. In any case, output data may be available from the clinical trial. In this way, the system 100 is able to make use of information (e.g. output data) relating to clinical trials that are no longer active (i.e. in progress), about which significant and useful data may be available, such as success rates of therapies, effectiveness measures of therapies, and side effects associated with therapies. By using information about clinical trials which have provided output data (e.g. trials which have ended or completed), a clinician is able to consider clinical trials for therapies which their subject was not able to participate, even though they may meet the inclusion criteria. For example, such a clinical trial may have started (and may have been completed) before the subject was diagnosed with the particular medical condition for which the clinical trial may be relevant.

As noted above, the processor 102 may be configured to identify the set of clinical trials by searching a clinical trails database. In some embodiments, the clinical trials database may include at least clinical trials that have provided output data. For example, the clinical trials database may include trials that have ended. The clinical trials database may also include clinical trials which are active or ongoing.

According to some embodiments, the processor 102 may be configured to rank the therapies using a two-step process. In a first step, the processor 102 may rank the clinical trials identified. This may be considered to be a "coarse", or broad ranking step. In a second step, the processor 102 may rank the therapies according to a fine ranking, or "fine-tuning" step, as discussed below.

Thus, in order for the processor 102 to rank the therapies, the processor may be configured to rank the identified set of clinical trials. In some embodiments, the identified set of clinical trials may be ranked based on clinical benefits achieved by each clinical trial. For example, the clinical trials may be ranked in order of the clinical benefits they achieve. The clinical benefits achieved by a clinical trial may be defined by at least one of: a measure of the progression-free survival of participants in the clinical trial; a measure of the overall survival of participants in the clinical trial; and a measure of the response rate of participants in the clinical trial. The measure of the progression-free survival of a subject is the length of time during and after the treatment of a disease by a therapy (e.g. in a clinical trial) that a subject (e.g. a participant in a clinical trial) lives with the disease without the disease getting worse. The measure of the overall survival is the length of time during and after treatment for a disease by a therapy that a subject is still alive. The measure of the response rate is the proportion of participants exhibiting a defined positive response (e.g. a reduction of a defined amount in the size of a tumor) over a defined period. In some embodiments, the achieved clinical benefits may be measured as a comparison to the response from the standard of care (i.e. the standard recommended treatment). In other embodiments, other measures of the clinical benefits of therapies may be used to rank the clinical trials.

In some embodiments, the processor 102 may, in addition to or as an alternative to the clinical trial ranking above, be configured to rank the identified set of clinical trials according to at least one of: a similarity of features of the subject to corresponding features of participants of the clinical trials; and a level of information available regarding the clinical trials. Thus, if the subject and a large number of participants in a particular clinical trial have a significant number of features (e.g. features included in the subject profile and in profiles associated with the trial participants) in common with one another, then that particular clinical trial may be ranked higher than a clinical trial in which the participants generally have fewer features in common with the subject. For some clinical trials, large amounts of information may be available (e.g. may have been published following the conclusion of the trial) regarding the trial itself and regarding the participants of the trial. However, in some scenarios, relatively little information may be available regarding a trial and its participants. Thus, the difference in the amount of information (or, the granularity of information) available for the clinical trials may be taken into account in the ranking. For example, a clinical trial for which a large amount of information is available may be ranked higher than a clinical trial for which relatively little information is available.

From the various clinical trial ranking techniques discussed above, an initial (coarsely) ranked list of clinical trials may be obtained. This list may include clinical trials which, while relevant to a particular subject in terms of the criteria used for ranking above, may not be suitable for the subject for other reasons. Thus, the initial list may be further adapted by the processor 102 to obtain a more relevant list from which therapies may be selected for a subject. It is noted that the steps of ranking the clinical trials discussed above (i.e. the coarse ranking) may not be performed. In some embodiments, the processor 102 may deduce the therapies that correspond to the clinical trials in the list and rank the therapies using techniques discussed below, without first ranking the clinical trials.

In some embodiments, the processor 102 may be configured to rank the therapies based on a weighting score. For example, some therapies may be considered more likely to give rise to an effective outcome than others. The weighting score may, in some embodiments, be based on one or more of: a satisfaction score, an outcome score and a supplemental effect score.

A satisfaction score may indicate a degree of satisfaction of the inclusion criteria by the plurality of features of the subject profile. Thus, a therapy corresponding to a clinical trial which has a relatively large number of inclusion criteria that are met by the subject (i.e. features in the subject profile satisfy a large number of the inclusion criteria) is given a relatively high weighting score.

An outcome score may indicate an extent to which each trial achieved clinical benefits. Thus, a relatively high weighting score may be given to a therapy if the corresponding clinical trial achieved strong clinical benefits. As discussed above, the clinical benefits may be defined by one or more of: a measure of the progression-free survival of participants in the clinical trial; a measure of the overall survival of participants in the clinical trial; and a measure of the response rate of participants in the clinical trial.

A supplemental effect score may indicate a measure of expected side effects caused by each trial. Such side effects may include adverse events that occur as a result or consequence of a therapy under trial. The side effects may be measured in terms of frequency and/or severity. For example, a relatively low weighting score may be given to a therapy which, from its corresponding clinical trial, is seen to cause frequently-occurring significant side effects. The supplemental effect score may be referred to as a toxicity score or a toxicity measure, as such a score relates to the toxicity and/or harmful effects that could be caused to a subject.

Following the "fine tuning" step of the ranking process, the ranked therapies may be suitable for review by a clinician, and ranked in such a way that a clinician may be able to determine quickly which therapy or therapies are most suitable for a particular subject, and are likely to have the most positive clinical effect on the subject.

However, in some embodiments, the processor 102 may be configured to perform further processing on the ranked therapies in order to produce an even more relevant list of therapies. The processor 102 may be configured to group the ranked therapies according to at least one of: biological similarities between the therapies; and similarities between features of the subject profile and features of profiles of participants in the set of clinical trials. Thus, within the ranked list, therapies which have biological similarities (e.g. therapies which use the same drug, or therapies which target the same part of the body) may be grouped together. Similarly, therapies which have corresponding clinical trials in which a large number of participants share common features (e.g. features in their profiles) with the subject may be grouped together. By grouping together certain therapies in the linked list, a clinician may quickly be able to determine those therapies and/or those clinical trials which are most relevant to the subject, and the reason or reasons for the relevance.

In some embodiments, the processor 102 may be configured to identify therapies having corresponding clinical trials for which the subject nearly met the inclusion criteria, but fell short by less than a threshold amount (e.g. missed only one of the inclusion criteria). In this way, the processor 102 may identify clinical trials which are relevant to the subject but which the subject could not officially participate due to the failure to meet, for example, one relatively insignificant requirement. A subject who is so close to meeting the inclusion criteria might be expected to respond to the therapy in a manner similar to the participants of the corresponding clinical trial. Such a clinical trial may correspond to a therapy that is helpful to the subject but which would otherwise have not been considered by the clinician.

The processor may, in some embodiments, be configured to remove one or more therapies from the ranked therapies if they fail to meet a particular defined criteria, or defined requirement. For example, a particular therapy may be removed from the ranked list of therapies if it is considered to be unsuitable for the subject, based on the subject profile. Such a scenario may occur, for example, if the therapy requires the application of a particular drug, but the subject profile indicates that the subject is allergic to the drug.

Once the list of therapies has been ranked, it may be displayed to a clinician. The processor 102 may be configured to present the output ranked therapies to a user. The user may, for example, be a clinician. The ranked therapies may be presented along with at least one of: expected benefits of each presented therapy; and potential risks associated with each presented therapy. In this way, the person viewing the presented list is able to determine quickly the potential strengths of the therapies in terms of the clinical benefits that may be expected from each therapy, and the potential weaknesses of the therapies in terms of the potential risks that may result from each therapy. The ranked therapies may be displayed on a display device associated with the system 100.

Fig. 2 shows an example of the system 100, comprising the processor 102, along with a number of additional, optional components. The system 100 of Fig. 2 may comprise a user interface 202 and/or a display 204. The user interface 202 may be configured for receiving a user input, such as data representing a subject profile, or clinical trial data. The display 204 may be configured for presenting the ranking therapies to a user. The user interface 202 and/or the display may be controlled by the processor 102.

The system 100 of Fig. 2 may be connected to a clinical trials database 206, which may store clinical trials data for use by the processor 102. The system 100 of Fig. 2 may also be connected to a clinical database 208, which may store clinical data relating to one or more subjects (i.e. subjects) for use by the processor 102. The clinical trials database 208 may include a description of each clinical trial, details, of the inclusion criteria for each trial, the composition of the participants of each trial, information (e.g. profiles) relating to participants of each trial, the treatment arms of each trial and the distribution of participants over the arms of the trial, and the achieved clinical benefits relative the standard of care for each arm of the trial. It will be appreciated that the connections to the databases 206, 208 may be made using wired or wireless connections, and the databases may be stored in storage media within the system 100, or remote from the system.

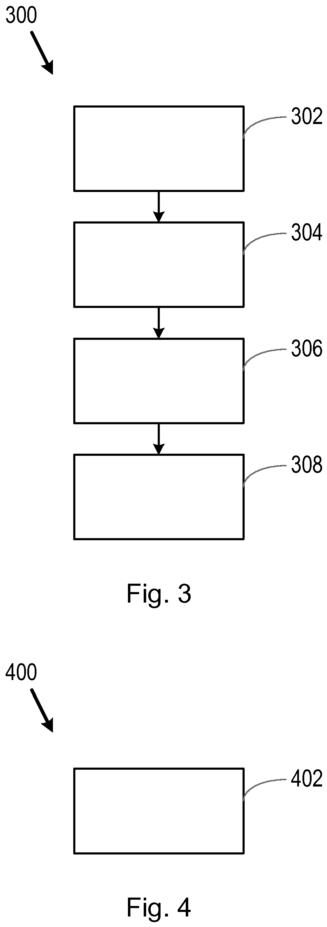

According to another aspect, a method (e.g. a computer-implemented method) is provided. The method may be a method for ranking a plurality of therapies for a subject. Steps of the method may be performed by the processor 102, or by another processing device. Fig. 3 is a flowchart of an example of a method 300 for ranking a plurality of therapies for a subject. The method comprises, at step 302, establishing a subject profile for defining the subject according to a plurality of features. At step 304, the method 300 comprises identifying a set of clinical trials that are relevant to the subject, wherein each clinical trial in the set of relevant clinical trials corresponds to at least one therapy and has inclusion criteria that are satisfied by features of the plurality of features of the subject profile. The method comprises, at step 306, ranking the therapies corresponding to the relevant clinical trials. At step 308, the method 300 comprises outputting the ranking of the therapies for the subject. In general, the method may comprise any of the steps performed by the processor 102 discussed above.

Fig. 4 is a flowchart of an example of a method 400 for ranking a plurality of therapies for a subject. The method 400 may include steps of the method 300. The method 400 may further comprise, at step 402, applying a weighting score to each therapy. In some embodiments, the ranking may be based on the applied weighting score. In this way, as discussed above, more factors may be taken into account in the assessment of the possible therapies available for a clinician to choose for their subject.

In some embodiments, the weighting score may be based on at least one of a satisfaction score indicating a degree of satisfaction of the inclusion criteria by the plurality of features of the subject profile; an outcome score indicating an extent to which each trial achieved clinical benefits; and a supplemental effect score indicating a measure of expected side effects caused by each trial.

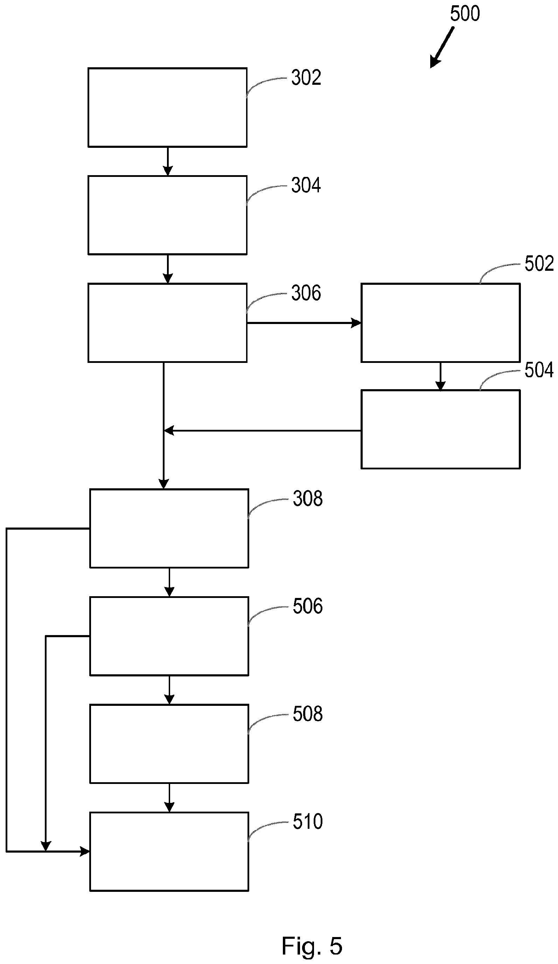

Fig. 5 is a flowchart of an example of a method 500 for ranking a plurality of therapies for a subject. The method 500 may include the steps of establishing a subject profile (step 302), identifying a set of clinical trials (step 304), and ranking the therapies (step 306). The step of ranking 306 the therapies may, in some embodiments, comprise, at step 502, ranking the identified set of clinical trials based on clinical benefits achieved by each clinical trial. As noted above, the clinical benefits achieved by a clinical trial may be defined by at least one of: a measure of the progression-free survival of participants in the clinical trial; a measure of the overall survival of participants in the clinical trial; and a measure of the response rate of participants in the clinical trial.

In some embodiments, the method 500 may further comprise, at step 504, ranking the identified set of clinical trials according to at least one of: a similarity of features of the subject to corresponding features of participants of the clinical trials; and a level of information available regarding the clinical trials. The method 500 may continue by outputting (step 308) the ranking of the therapies. In embodiments in which the clinical trials are not ranked (i.e. steps 502 and 504 are not performed), the method 500 may proceed to outputting the ranking (step 306).

By ranking the clinical trials according to steps 502 and 504, a user (e.g. a clinician) may obtain a list of therapies which might be suitable for a subject, the having been coarsely ordered in terms of relevance, based on the factors considered in steps 502 and 504.

At step 506, the method 500 may comprise ranking the therapies based on a weighting score. The weighting score may be based on at least one of: a satisfaction score indicating a degree of satisfaction of the inclusion criteria by the plurality of features of the subject profile; an outcome score indicating an extent to which each trial achieved clinical benefits; and a supplemental effect score indicating a measure of expected side effects caused by each trial.

The method 500 may optionally comprise, at step 508, grouping the ranked therapies according to at least one of: biological similarities between the therapies; and similarities between features of the subject profile and features of profiles of participants in the set of clinical trials.

The output ranked therapies may, at step 510, be presented to a user. The therapies may be presented (at step 510) at one or more occasions during the method, such as: i) in the order in which they are output at step 308; ii) in the order in which they are output following their ranking based on the weighting score at step 506; or iii) in the order in which they are output following the grouping at step 508. In some embodiments, the therapies may be presented (at step 510) along with at least one of: expected benefits of each presented therapy; and potential risks associated with each presented therapy. In some embodiments, a user may be provided with an opportunity to manually adapt one or more of the inclusion criteria of a clinical trial to see what effect such a change has on the outputted list. For example, a user might relax a criterion, such as an age range, to see which additional trials and corresponding therapies are added to the list. This may have particular benefits if the list presented at step 510 is particularly short, for example.

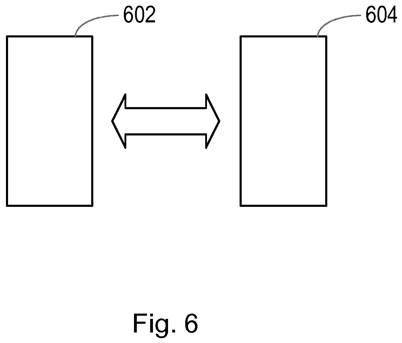

According to another aspect, a computer program product is provided. Fig. 6 is a simplified schematic of a computer readable medium 602 and a processor 604. According to some embodiments, a computer program product comprises a non-transitory computer readable medium 602, the computer readable medium having computer readable code embodied therein, the computer readable code being configured such that, on execution by a suitable computer or processor 604, the computer or processor is caused to perform any of the methods 300, 400, 500 disclosed herein. The processor 604 may comprise, or be similar to, the processor 102.

The processor 102, 604 can comprise one or more processors, processing units, multi-core processors or modules that are configured or programmed to control apparatus and/or the system 100 in the manner described herein. In particular implementations, the processor 102, 604 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A clinical decision support system (100), comprising:
a processor (102) configured to:
establish a subject profile for defining a subject according to a plurality of features;
identify a set of clinical trials that are relevant to the subject, wherein each clinical trial in the set of relevant clinical trials corresponds to at least one therapy and has inclusion criteria that are satisfied by features of the plurality of features of the subject profile;
rank the therapies corresponding to the relevant clinical trials; and
output the ranking of the therapies for the subject.

2. A clinical decision support system (100) according to claim 1, wherein, in order to rank the therapies, the processor (102) is configured to:
rank the identified set of clinical trials based on clinical benefits achieved by each clinical trial.

3. A clinical decision support system (100) according to claim 2, wherein the processor (102) is configured to:
rank the identified set of clinical trials according to at least one of: a similarity of features of the subject to corresponding features of participants of the clinical trials; and a level of information available regarding the clinical trials.

4. A clinical decision support system (100) according to claim 2 or claim 3,
wherein the clinical benefits achieved by a clinical trial are defined by at least one of: a measure of the progression-free survival of participants in the clinical trial; a measure of the overall survival of participants in the clinical trial; and a measure of the response rate of participants in the clinical trial.

5. A clinical decision support system (100) according to any of the preceding claims, wherein the processor (102) is configured to :
rank the therapies based on a weighting score.

6. A clinical decision support system (100) according to claim 5, wherein the weighting score is based on at least one of:
a satisfaction score indicating a degree of satisfaction of the inclusion criteria by the plurality of features of the subject profile;
an outcome score indicating an extent to which each trial achieved clinical benefits; and
a supplemental effect score indicating a measure of expected side effects caused by each trial.

7. A clinical decision support system (100) according to any of the preceding claims, wherein the processor (102) is configured to:
establish the subject profile by obtaining a clinical information relating to the subject from a clinical database (208).

8. A clinical decision support system (100) according to any of the preceding claims, wherein the processor (102) is configured to:
identify the set of clinical trials by searching a clinical trails database (206), the clinical trials database including at least clinical trials that have provided outcome data.

9. A clinical decision support system (100) according to any of the preceding claims, wherein the processor (102) is configured to:
group the ranked therapies according to at least one of: biological similarities between the therapies; and similarities between features of the subject profile and features of profiles of participants in the set of clinical trials.

10. A clinical decision support system (100) according to any of the preceding claims, wherein the processor (102) is configured to:
present the output ranked therapies to a user along with at least one of: expected benefits of each presented therapy; and potential risks associated with each presented therapy.

11. A clinical decision support system (100) according to any of the preceding claims, wherein a clinical trial is relevant to the subject if a threshold number of inclusion criteria are satisfied by features of the subject profile.

12. A method (300) for ranking a plurality of therapies for a subject, the method comprising:
establishing (302) a subject profile for defining the subject according to a plurality of features;
identifying (304) a set of clinical trials that are relevant to the subject, wherein each clinical trial in the set of relevant clinical trials corresponds to at least one therapy and has inclusion criteria that are satisfied by features of the plurality of features of the subject profile;
ranking (306) the therapies corresponding to the relevant clinical trials; and
outputting (308) the ranking of the therapies for the subject.

13. A method (300, 400) according to claim 12, further comprising:
applying (402) a weighting score to each therapy;
wherein the ranking (306) is based on the applied weighting score.

14. A method (300, 400) according to claim 13, wherein the weighting score is based on at least one of:
a satisfaction score indicating a degree of satisfaction of the inclusion criteria by the plurality of features of the subject profile;
an outcome score indicating an extent to which each trial achieved clinical benefits; and
a supplemental effect score indicating a measure of expected side effects caused by each trial.

15. A computer program product comprising a non-transitory computer readable medium (602), the computer readable medium having computer readable code embodied therein, the computer readable code being configured such that, on execution by a suitable computer or processor (604), the computer or processor is caused to perform the method of any of claims 12 to 14.
